# EUROPÄISCHE PATENTSCHRIFT

(11) **EP 2 947 139 B1**
(45) Veröffentlichungstag und Bekanntmachung des Hinweises auf die Patenterteilung: **07.09.2016**
(21) Anmeldenummer: 15152893.2
(22) Anmeldetag: 28.01.2015
(51) Int. Cl.: C12M 1/00, C12M 1/12

(54) **Reaktor, Reinigungsverfahren für diesen sowie Verwendung**
Reactor, cleaning method for same, and use
Réacteur, son procédé de nettoyage et utilisation

(30) Priorität: 25.04.2014 DE 102014005889; 23.07.2014 DE 102014010749
(43) Veröffentlichungstag der Anmeldung: 25.11.2015
(73) Patentinhaber: Infors AG, 4103 Bottmingen (CH)
(72) Erfinder: Hawrylenko, Alexander, 4103 Bottmingen (CH); Cueni, Dieter, 4222 Zwingen (CH)
(74) Vertreter: Toleti, Martin

(56) Entgegenhaltungen:
- EP-A2- 1 632 562
- WO-A2-2009/051478
- US-A1- 2003 073 231

## Beschreibung

### Technisches Gebiet

Die vorliegende Erfindung betrifft einen Reaktor gemäß dem Oberbegriff des Anspruchs 1, nämlich einen Reaktor zur photochemischen Züchtung einer flüssigen Kultur von Klein- und Kleinstlebewesen, insbesondere von Algen, mit einem Tank, mit einem daran über eine Pumpe angeschlossenen Vorlauf sowie Rücklauf, welche Pumpe im Betrieb mit einer vorgebbaren Nenndrehzahl in einer die Fließrichtung der Kultur bewirkenden Drehrichtung umläuft, und mit mehreren vom Vorlauf bei Betrieb in Fließrichtung auf den Rücklauf zu gespeisten, jeweils paarweise hintereinander über Verbindungsstücke miteinander verbundenen Rohren, wobei die Kultur der Lichtstrahlung einer einen Außenmantel aufweisenden stabförmigen Lampe für die photochemische Züchtung ausgesetzt ist, welcher symmetrisch zu und in jedem Rohr unter Bildung eines Ringraums angeordnet ist, und wobei in jedem Rohr ein Reinigungsteil angeordnet ist, das zwecks Reinigung des Außenmantels längs diesem verschiebbar ist und dessen radiale Innenseite an den Außenmantel form- sowie größenmäßig angepasst ist.

Des Weiteren betrifft die vorliegende Erfindung ein Reinigungsverfahren für den Reaktor sowie eine Verwendung.

### Stand der Technik

Reaktoren sind in einer Vielzahl von Ausführungsformen bekannt (GB 2 118 572 A).

Von Nachteil bei diesem bekannten sowie auch bei anderen Reaktoren ist die Tatsache, dass sich die gezüchteten und im Kreislauf geführten Kulturen letztlich mit der Folge absetzen, dass das für die Züchtung notwendige Licht in einem Maße gedämpft wird, dass die Züchtung mit gutem Wirkungsgrad nicht mehr möglich ist.

Aus diesem Grunde muss die Anlage angehalten und die Züchtung der Kultur unterbrochen oder abgebrochen werden, weil die Rohre von ihrem inneren Belag aus abgestorbener Kultur befreit werden müssen. Um dies richtig durchführen zu können, muss die Anlage im Bereich der Rohrführung jedenfalls zerlegt und im zerlegten Zustand gereinigt werden, was aufwändig ist und zu längeren Unterbrechungen führt, was in hohem Maße unerwünscht ist.

Um die Reinigung zu vereinfachen, ist schon ein gattungsgemäßer Reaktor nach dem Oberbegriff des Anspruchs 1 bekannt geworden (EP 1 632 562 A2). Bei diesem Reaktor erfolgt die Reinigung entweder manuell mit einem Schieber, der am Reinigungsteil angreift, wozu jedoch der Reaktor im Bereich der Rohre geöffnet werden, was aufwändig ist, oder das Reinigungsteil wird ohne Öffnen des Reaktors von außen über geeignet vorgesehene Magnete bewegt, wobei der Außenmantel der Lampe gereinigt wird, was jedoch einen komplizierteren Aufbau voraussetzt.

### Darstellung der vorliegenden Erfindung: Aufgabe, Lösung, Vorteile

Ausgehend von den vorstehend dargelegten Nachteilen und Unzulänglichkeiten sowie unter Würdigung des umrissenen Stands der Technik liegt der vorliegenden Erfindung die Aufgabe zugrunde, einen gattungsgemäßen Reaktor gemäß dem Oberbegriff des Anspruchs 1 so auszugestalten, dass eine einfachste Reinigung ohne die Notwendigkeit der Zerlegung des Reaktors möglich ist.

Diese Aufgabe wird bei einem gattungsgemäßen Reaktor gemäß dem Oberbegriff des Anspruchs 1 erfindungsgemäß durch dessen kennzeichnende Merkmale gelöst, also dadurch
- dass beide Enden eines jeden Rohrs und/oder die Verbindungsstücke eine Querschnittserweiterung gegenüber dem Ringraum aufweisen,
- dass jedes Rohr einen längs des Außenmantels bewegbaren Schlitten aufweist,
- dass für jeden Schlitten im Bereich der Querschnittserweiterung ein Endanschlag vorgesehen ist,
- dass jeder Schlitten kraftschlüssig mit dem Reinigungsteil verbunden ist,
- dass der Schlitten mit einem verschiebbaren Kolben versehen ist und
- dass der Kolben im Bereich außerhalb der Querschnittserweiterung den Ringraum ausfüllt.

Außerdem wird diese Aufgabe durch das Reinigungsverfahren gemäß Anspruch 12, also dadurch
- dass der Kolben eines jeden Schlittens im Züchtungsbetrieb bei Nenndrehzahl der Pumpe in der einen Drehrichtung der Pumpe in einer ersten Fließrichtung der Kultur im Bereich der Querschnittserweiterung in seiner einen Endlage, nämlich am ersten Anschlag angeordnet ist,
- dass bei Reversierung der Drehrichtung der Pumpe, unter Bewirkung einer zur ersten Fließrichtung reversierten oder umgekehrten zweiten Fließrichtung der Kolben eines jeden Schlittens bei dessen Stillstand von seinem ersten Anschlag zur Anlage an seinem zweiten Anschlag verschoben wird,
- wobei danach zwecks Reinigung jeder Schlitten zusammen mit seinem Kolben in der zweiten Fließrichtung der Kultur durch den Ringraum bis zum Endanschlag an der anderen Seite eines jeden Rohrs verschoben wird,
- dass nach erneuter Umkehr der Fließrichtung alle Kolben von der Anlage am zweiten Anschlag zur Anlage am ersten Anschlag verschoben werden und
- dass danach die Schlitten zusammen mit den Kolben durch den Ringraum, insbesondere unter erneuter Reinigung, bis zum Erreichen des Endanschlags am anderen Ende eines jeden Rohrs in die Ausgangslage zurück bewegt werden,
sowie durch die Verwendung gemäß Anspruch 15 gelöst, also durch die Verwendung einer Querschnittserweiterung mit einem längs des Außenmantels bewegbaren, kraftschlüssig mit dem Reinigungsteil verbundenen Schlitten in jedem Rohr, welcher Schlitten mit einem auf seinem Mantel zwischen zwei Anschlägen verschiebbaren Kolben versehen ist.

Ein mit solchen erfindungsgemäßen Merkmalen versehener Reaktor sowie ein entsprechendes Verfahren bewirken, dass der in jedem Rohr in den Bereich der einen Querschnittserweiterung zurück gebrachte Schlitten am Endanschlag und der auf ihm befindliche Kolben in seiner einen Endlage, nämlich am ersten Anschlag verbleibt, wonach die Pumpe in die eine Drehrichtung zum Zwecke der Züchtung der Klein- und Kleinstlebewesen im Betriebszustand umgesteuert und damit die Fließrichtung der Kultur reversiert wird.

In dieser Stellung und im Betrieb bei Nenndrehzahl in dieser einen Drehrichtung der Pumpe verbleibt sowohl der Schlitten am Endanschlag als auch der auf ihm vorgesehene Kolben in seiner Endlage, dem ersten Anschlag, im Bereich der Erweiterung und wird trotz der Strömung der flüssigen Kultur von dieser nicht weg bewegt, weil diese den Kolben gewissermaßen widerstandsfrei jedenfalls dann umfließt, wenn die Querschnittsfläche der Querschnittserweiterung mindestens jener des Ringraums zwischen dem Außenmantel der stabförmigen Lampe und dem diese umgebenden Rohr entspricht.

Bei beibehaltener gleichbleibender Drehrichtung und Erhöhung, zum Beispiel Verdoppelung, der Nenndrehzahl der Pumpe wird allerdings aufgrund der durch die schnellere Strömung bewirkten Kraft zunächst der Kolben jeden Schlittens von seinem Betriebsanschlag in Richtung des zweiten Anschlags, dem Reinigungsanschlag, bis zur Anlage an diesem bewegt, wonach der Schlitten und der Kolben in Fließrichtung zusammen bewegt werden.

Hierbei wird der Kolben aus dem Bereich der Querschnittserweiterung in den von ihm ausgefüllten Ringraum hineingeschoben und - jetzt als Kolben in einem Zylinder wirkend - durch diesen hindurch gedrückt, wobei das mit dem Schlitten kraftschlüssig verbundene Reinigungsteil mit seiner an den Außenmantel der stabförmigen Lampe form- sowie größenmäßig angepassten radialen Innenseite am Außenmantel unter dessen gleichzeitiger Reinigung bis zum Ende des Rohrs entlang bewegt wird und schließlich dort anhält, weil es in den dortigen Bereich der Querschnittserweiterung gelangt.

Danach wird die Drehrichtung der Pumpe und damit die Fließrichtung umgekehrt. Hierbei wird zunächst der Kolben bei stehendem Schlitten von seinem Reinigungsanschlag bis zur Anlage an seinem Betriebsanschlag am Schlitten zurückverschoben, wonach dieser Schlitten bis an den Endanschlag im Bereich der Querschnittserweiterung mit in die eingangs geschilderte Stellung als Ausgangslage zurück geschleppt wird.

Läuft nach erneuter Reversierung die Pumpe mit Nenndrehzahl, so kann die Züchtung der Kultur ohne einen sich anschließenden Reinigungsschritt weitergeführt werden.

Die erfindungsgemäße Ausbildung des Reaktors ist einfach, und mit ihr kann dessen einfachste Reinigung ohne die Notwendigkeit der Zerlegung durch einfache Reversierung und Erhöhung der Drehzahl der Pumpe für die flüssige Kultur in überraschend sowie verblüffend einfacher Weise bewirkt werden. Das erfindungsgemäße Prinzip mit den geschilderten Vorteilen ist ohne Vorbild im Stand der Technik.

Weitere vorteilhafte Ausgestaltungen und zweckmäßige Weiterbildungen der vorliegenden Erfindung sind in den Nebenansprüchen sowie in den Unteransprüchen gekennzeichnet; insbesondere
- kann der Endanschlag als in den Bewegungsweg des Kolbens reichende, am Verbindungsstück und/oder am Rohr festgelegte Platte ausgebildet sein und/oder
- kann jeder Schlitten auf seinem Mantel zwischen einem ersten Anschlag und einem zweiten Anschlag bewegbar sein und/oder
- kann der erste Anschlag und/oder der zweite Anschlag als radial nach außen stehender Vorsprung ausgebildet sein und/oder
- kann der erste Anschlag am dem Tank näher gelegenen Ende eines jeden Schlittens vorgesehen sein und/oder
- kann der zweite Anschlag am der Pumpe näher gelegenen Ende eines jeden Schlittens vorgesehen sein und/oder
- kann der Schlitten an seinem radial innen liegenden Bereich form- sowie größenmäßig an den Außenmantel angepasst sein und/oder
- kann der Kolben als Ringscheibe ausgebildet sein und/oder
- kann der Kolben form- sowie größenmäßig an den Ringraum zu dessen Ausfüllung angepasst sein und/oder
- kann die Querschnittserweiterung zumindest teilweise im Bereich des Verbindungsstücks vorgesehen sein und/oder
- kann die Größe des Querschnitts der Querschnittserweiterung zumindest der Größe des Querschnitts des Ringraums entsprechen und/oder
- kann das Reinigungsteil als mindestens ein an der radialen Innenseite des Schlittens und an dessen beiden Enden vorgesehener O-Ring ausgebildet sein und/oder
- kann bei Reversierung der Drehrichtung der Pumpe und/oder bei Umkehr der Fließrichtung die Drehzahl der Pumpe erhöht werden und/oder
- kann nach Erreichen des Endanschlags die erhöhte Drehzahl der Pumpe wieder auf die die erste, insbesondere normale, Fließrichtung und eine normale Strömungsgeschwindigkeit bewirkende Nenndrehzahl abgesenkt werden.

### Kurze Beschreibung der Zeichnungen

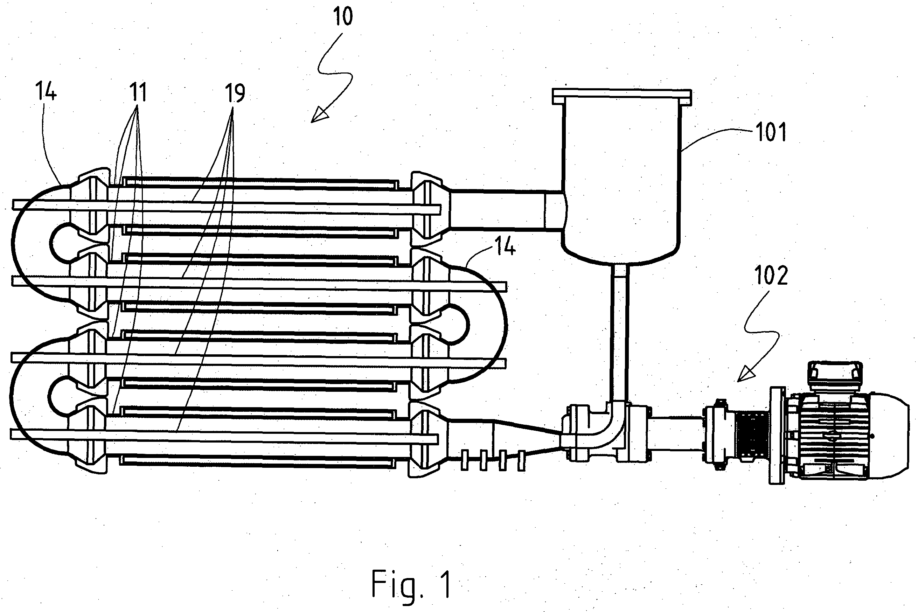

Wie bereits vorstehend erörtert, gibt es verschiedene Möglichkeiten, die Lehre der vorliegenden Erfindung in vorteilhafter Weise auszugestalten und weiterzubilden. Hierzu wird einerseits auf die dem Anspruch 1 nachgeordneten Ansprüche verwiesen, andererseits werden weitere Ausgestaltungen, Merkmale und Vorteile der vorliegenden Erfindung nachstehend unter anderem anhand des durch Fig. 1 bis Fig. 5 veranschaulichten Ausführungsbeispiels näher erläutert.

Es zeigt:
- Fig. 1: in schematischer Ansicht ein Ausführungsbeispiel für einen Reaktor gemäß der vorliegenden Erfindung, der nach dem Verfahren gemäß der vorliegenden Erfindung arbeitet;
- Fig. 2: in schematischer Ansicht den Reaktor gemäß Fig. 1 in einer ersten Stellung (Betriebsstellung oder Fließrichtung der Kultur);
- Fig. 3: in teilweise abgebrochener Querschnittsansicht den Reaktor aus Fig. 2;
- Fig. 4: in schematischer Ansicht den Reaktor gemäß Fig. 1 in einer zweiten Stellung (Reinigungsstellung); und
- Fig. 5: in teilweise abgebrochener Querschnittsansicht den Reaktor aus Fig. 4.

Gleiche oder ähnliche Ausgestaltungen, Elemente oder Merkmale sind in Fig. 1 bis Fig. 5 mit identischen Bezugszeichen versehen.

### Bester Weg zur Ausführung der vorliegenden Erfindung

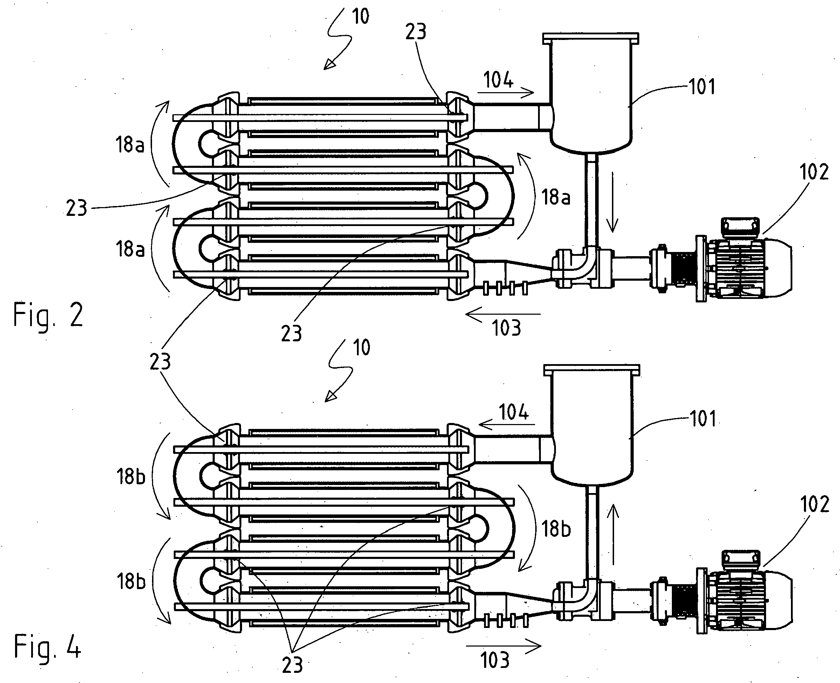

Der in Fig. 1, in Fig. 2 und in Fig. 4 schematisch dargestellte Reaktor 10 dient zur photochemischen Züchtung einer flüssigen Kultur von Klein- und Kleinstlebewesen, vorzugsweise von Algen, und entspricht im Übrigen jenem der Gattung (EP 1 632 562 A2).

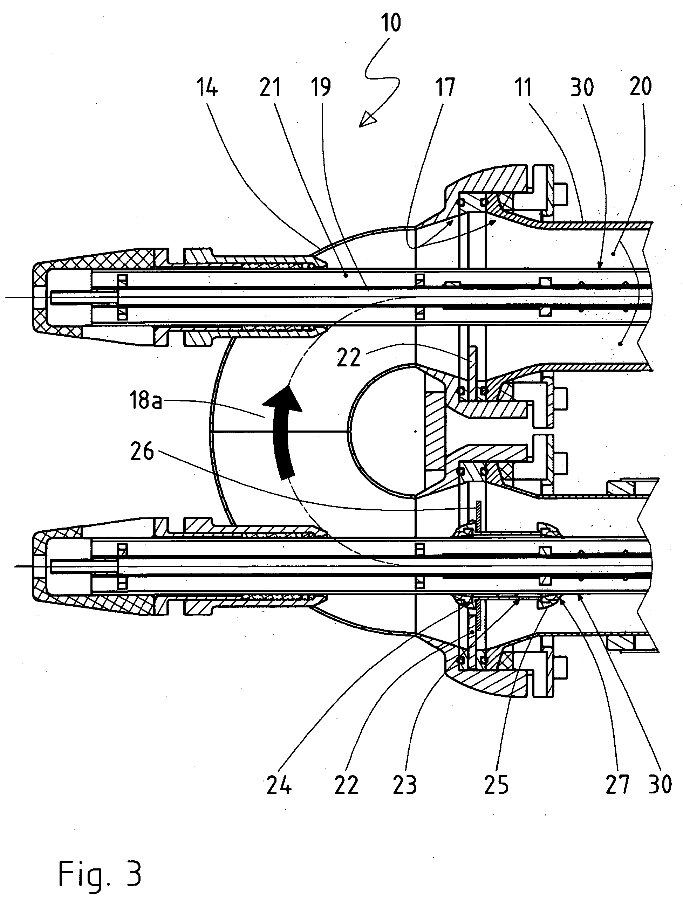

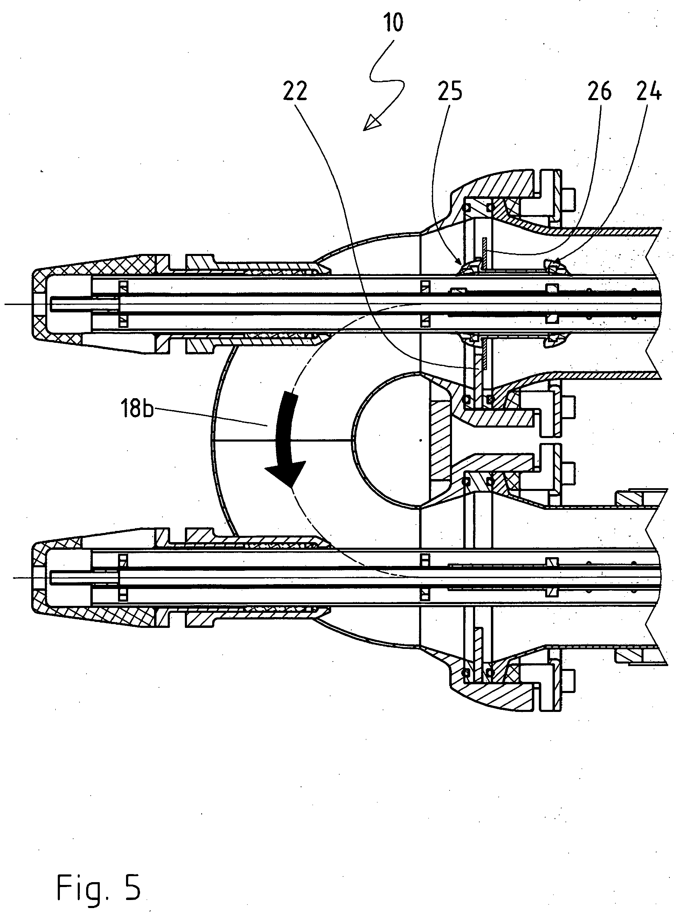

Der Reaktor 10 ist mit einem Tank 101, mit einem daran über eine Pumpe 102 angeschlossenen Vorlauf 103 (vgl. Fig. 2 und Fig. 4) sowie mit einem Rücklauf 104 (vgl. Fig. 2 und Fig. 4) versehen. Die Pumpe 102 läuft im Betrieb mit einer vorgebbaren Nenndrehzahl in einer die Fließrichtung 18a (vgl. Fig. 2 und Fig. 3) bzw. 18b (vgl. Fig. 4 und Fig. 5) der Kultur bewirkenden Drehrichtung um.

Der Reaktor 10 weist mehrere bei Betrieb in Fließrichtung 18a (vgl. Fig. 2 und Fig. 3) vom Vorlauf 103 auf den Rücklauf 104 zu gespeiste, parallel zueinander verlaufende Rohre 11 auf. Diese Rohre 11 sind jeweils paarweise an benachbarten Enden über ein Verbindungsstück 14 miteinander unter Bewirkung eines mäanderförmigen Strömungsverlaufs der flüssigen Kultur durch die Rohre 11 verbunden.

Die Kultur ist der Lichtstrahlung einer stabförmigen Lampe 19 für die photochemische Züchtung ausgesetzt. Hierbei mündet das letzte Rohr 11 in den Rücklauf 104, der zum Tank 101 führt, von dem die Pumpe 102 über den Vorlauf 103 zum ersten Rohr 11 in Fließrichtung 18a verläuft, wie schematisch in Fig.2 dargestellt. Fig.4 zeigt dieselbe Darstellung, jedoch in einer zweiten Betriebslage, der Reinigungsstellung, des Reaktors 10.

Der in Fig. 3 dargestellte Teil ist eine Detailansicht des in Fig. 2 links gezeigten Bereichs des Reaktors 10; entsprechend ist der in Fig. 5 dargestellte Teil eine Detailansicht des in Fig. 4 links gezeigten Bereichs des Reaktors 10:
In jedem Rohr 11 ist konzentrisch zum kreiszylindrischen Rohr 11 mindestens eine stabförmige Lampe 19 als Lichtquelle so angeordnet, dass deren Achsen sowohl parallel zueinander verlaufen als auch mit jener des kreiszylindrischen Rohrs 11 zusammenfallen. Die Lampe 19 weist eine eigentliche elektrische stabförmige Lampe und ein diese stabförmige Lampe symmetrisch sowie konzentrisch unter Bildung eines Ringraums 20 umgebendes Schutzrohr 21 mit zu reinigendem Außenmantel 30 auf.

Das Verbindungsstück 14 ist auf seiner Vorderseite mit zwei voneinander beabstandeten Öffnungen zur dichten Aufnahme des einen Endes jeweils eines Rohrs 11 von zwei zueinander benachbarten Rohren 11 versehen. Ferner ist das Verbindungsstück 14 im Inneren an dessen von den Rohren 11 abgewandter Rückseite mit zwei voneinander beabstandeten Ausnehmungen für die Lampen 19 versehen.

Jedes Ende eines jeden Rohrs 11 ist, wie aus Fig. 3 ersichtlich, mit einer gegenüber dessen Ringraum 20 vorgesehenen Querschnittserweiterung 17 versehen, die zumindest teilweise im Bereich des Verbindungsstücks 14 vorgesehen ist.

Jedes Rohr 11 weist einen längs des Außenmantels 30 der Lampe 19 bewegbaren, an seinem radial innen liegenden Bereich form- sowie größenmäßig an den Außenmantel 30 der Lampe 19 angepassten Schlitten 23 auf, der an einem Endanschlag 22 im Bereich der Querschnittserweiterung 17 zur Anlage kommt.

Jeder Schlitten 23 ist kraftschlüssig mit einem als an dessen radialer Innenseite vorgesehenen O-Ring ausgebildeten Reinigungsteil 27 verbunden.

Außerdem ist dem Schlitten 23 ein auf seinem Mantel als Ringscheibe ausgebildeter Kolben 26 zugeordnet. Im Bereich außerhalb der Querschnittserweiterung 17 ist der Kolben 26 in Form und Größe an den Ringraum 20 angepasst und füllt diesen aus.

Der Kolben 26 ist zwischen einem als radial nach außen stehender Vorsprung ausgebildeten ersten Anschlag 24 und einem als radial nach außen stehender Vorsprung ausgebildeten zweiten Anschlag 25 verschiebbar, wobei
- der erste Anschlag 24 am dem Tank 101 näher gelegenen Ende eines jeden Schlittens 23 und
- der zweite Anschlag 25 am der Pumpe 102 näher gelegenen Ende eines jeden Schlittens 23 angeordnet ist.

Der Endanschlag 22 ist als in den Bewegungsweg des Kolbens 26 reichende, am Verbindungsstück 14 und/oder am Rohr 11 festgelegte Platte ausgebildet.

Die Funktion des Reaktors 10 ist wie folgt:
Bei gleichbleibender Drehrichtung und Erhöhung, zum Beispiel Verdoppelung, der Nenndrehzahl der Pumpe 102 wird aufgrund der durch die Strömung in Fließrichtung 18a bewirkten Kraft zuvor der Kolben 26 des Schlittens 23 von seinem zweiten Anschlag 25 in Richtung des ersten Anschlags 24 bis zur Anlage am ersten Anschlag 24 bewegt, wonach der Schlitten 23 zusammen mit dem Kolben 26 in Fließrichtung 18a bis in die in Fig. 3 gezeigte Lage bewegt wird.

Hierbei wird der Kolben 26 aus dem von ihm ausgefüllten Ringraum 20 in den gezeigten Bereich der Querschnittserweiterung 17 bewegt und verbleibt am (End-)Anschlag 22. In dieser Lage, der Betriebsstellung, erfolgt die Züchtung der flüssigen Kultur, wobei in dieser Stellung, wie Fig. 2 schematisch zeigt, die Schlitten 23 im in Fließrichtung 18a hinteren und dem Tank 101 näheren Ende eines jeden Rohrs 11 angeordnet sind.

Zum Zweck der Reinigung wird die Pumpe 102 reversiert und damit die Fließrichtung von der ersten Richtung 18a (vgl. Fig. 2 und Fig. 3) in die zweite Richtung 18b (vgl. Fig. 4 und Fig. 5) umgekehrt, wobei optionalerweise die Drehzahl der Pumpe 102 und damit die Fließgeschwindigkeit erhöht werden kann.

Hierbei wird bei allen Schlitten 23 im Reaktor 10 zunächst der Kolben 26 - bei jeweils stehenden Schlitten 23 - von seinem ersten Anschlag 24 zu seinem zweiten Anschlag 25 bewegt (nicht gezeigt) und dann der Schlitten 23 zusammen mit dem Kolben 26 vom Bereich der Querschnittserweiterung 17 in den Ringraum 20 bewegt und durch diesen hindurch gedrückt.

Hierbei wird das mit dem Schlitten 23 kraftschlüssig verbundene Reinigungsteil 27 mit seiner an den Außenmantel 30 der Lampe 19 form- sowie größenmäßig angepassten radialen Innenseite am Außenmantel 30 unter dessen gleichzeitiger Reinigung bis zum Ende des (in Fig. 5 oberen) Rohrs 11 entlang bewegt und hält schließlich dort am (End-)Anschlag 22 an (vgl. Fig. 5). Die abgeschälten Verunreinigungen können bei dieser zweiten Fließrichtung 18b (vgl. Fig. 4 und Fig. 5) abtransportiert werden. Alle Schlitten 23 befinden sich in der in Fig. 4 gezeigten Lage.

Bei Beendigung des Reinigungsschritts wird die Fließrichtung der Kultur von der Richtung 18b (vgl. Fig. 4 und Fig. 5) wieder in die Richtung 18a (vgl. Fig. 2 und Fig. 3) geändert, wobei optionalerweise die Drehzahl der Pumpe 102 erhöht, zum Beispiel verdoppelt, werden kann.

Alle Kolben 26 werden bis zur Anlage an den ersten Anschlag 24 zurückverschoben, wonach der Schlitten 23 zu seiner Ausgangslage bis an den Endanschlag 22 im Bereich der Querschnittserweiterung 17 an der jeweils anderen Seite eines jeden Rohrs 11, gegebenenfalls unter erneuter Ablösung von Verunreinigungen, von den in Fig. 2 gezeigten Lagen in jene gemäß Fig. 4 zurückgeschleppt wird (nicht gezeigt) und dort verbleibt, so dass die Züchtung der Kultur ohne Reinigungsschritt weitergeführt werden kann.

### Liste der Bezugszeichen

- 10: Reaktor
- 11: Rohr
- 14: Verbindungsstück
- 17: Querschnittserweiterung
- 18a: erste Fließrichtung
- 18b: zweite, zur ersten Fließrichtung 18a reversierte oder umgekehrte oder entgegen gesetzte Fließrichtung
- 19: Lampe, insbesondere stabförmige Lampe
- 20: Ringraum
- 21: Schutzrohr
- 22: Endanschlag
- 23: Schlitten
- 24: erster Anschlag des Kolbens 26
- 25: zweiter Anschlag des Kolbens 26
- 26: Kolben des Schlittens 23
- 27: Reinigungsteil
- 30: Außenmantel der Lampe 19
- 101: Tank
- 102: Pumpe
- 103: Vorlauf
- 104: Rücklauf

## Patentansprüche

1. Reaktor (10) zur photochemischen Züchtung einer flüssigen Kultur von Klein- und Kleinstlebewesen, insbesondere von Algen, mit einem Tank (101), mit einem daran über eine Pumpe (102) angeschlossenen Vorlauf (103) sowie Rücklauf (104), welche Pumpe (102) im Betrieb mit einer vorgebbaren Nenndrehzahl in einer die Fließrichtung (18a) der Kultur bewirkenden Drehrichtung umläuft, und mit mehreren vom Verlauf (103) bei Betrieb in Fließrichtung (18a) auf den Rücklauf (104) zu gespeisten, jeweils paarweise hintereinander über Verbindungsstücke (14) miteinander verbundenen Rohren (11), wobei die Kultur der Lichtstrahlung einer einen Außenmantel (30) aufweisenden stabförmigen Lampe (19) für die photochemische Züchtung ausgesetzt ist, welcher symmetrisch zu und in jedem Rohr (11) unter Bildung eines Ringraums (20) angeordnet ist, und wobei in jedem Rohr (11) ein Reinigungsteil (27) angeordnet ist, das zwecks Reinigung des Außenmantels (30) längs diesem verschiebbar ist und dessen radiale Innenseite an den Außenmantel (30) form- sowie größenmäßig angepasst ist,
**dadurch gekennzeichnet,**
- **dass** beide Enden eines jeden Rohrs (11) und/oder die Verbindungsstücke (14) eine Querschnittserweiterung (17) gegenüber dem Ringraum (20) aufweisen,
- **dass** jedes Rohr (11) einen längs des Außenmantels (30) bewegbaren Schlitten (23) aufweist,
- **dass** für jeden Schlitten (23) im Bereich der Querschnittserweiterung (17) ein Endanschlag (22) vorgesehen ist,
- **dass** jeder Schlitten (23) kraftschlüssig mit dem Reinigungsteil (27) verbunden ist,
- **dass** der Schlitten (23) mit einem verschiebbaren Kolben (26) versehen ist und
- **dass** der Kolben (26) im Bereich außerhalb der Querschnittserweiterung (17) den Ringraum (20) ausfüllt.

2. Reaktor gemäß Anspruch 1, **dadurch gekennzeichnet, dass** der Endanschlag (22) als in den Bewegungsweg des Kolbens (26) reichende, am Verbindungsstück (14) und/oder am Rohr (11) festgelegte Platte ausgebildet ist.

3. Reaktor gemäß Anspruch 1 oder 2, **dadurch gekennzeichnet, dass** jeder Schlitten (23) auf seinem Mantel zwischen einem ersten Anschlag (24) und einem zweiten Anschlag (25) bewegbar ist.

4. Reaktor gemäß Anspruch 3, **dadurch gekennzeichnet, dass** der erste Anschlag (24) und/oder der zweite Anschlag (25) als radial nach außen stehender Vorsprung ausgebildet ist.

5. Reaktor gemäß Anspruch 3 oder 4, **dadurch gekennzeichnet,**
- **dass** der erste Anschlag (24) am dem Tank (101) näher gelegenen Ende eines jeden Schlittens (23) und/oder
- **dass** der zweite Anschlag (25) am der Pumpe (102) näher gelegenen Ende eines jeden Schlittens (23)
vorgesehen ist.

6. Reaktor gemäß mindestens einem der Ansprüche 1 bis 5, **dadurch gekennzeichnet, dass** der Schlitten (23) an seinem radial innen liegenden Bereich form- sowie größenmäßig an den Außenmantel (30) angepasst ist.

7. Reaktor gemäß mindestens einem der Ansprüche 1 bis 6, **dadurch gekennzeichnet, dass** der Kolben (26) als Ringscheibe ausgebildet ist.

8. Reaktor gemäß mindestens einem der Ansprüche 1 bis 7, **dadurch gekennzeichnet, dass** der Kolben (26) form- sowie größenmäßig an den Ringraum (20) zu dessen Ausfüllung angepasst ist.

9. Reaktor gemäß mindestens einem der Ansprüche 1 bis 8, **dadurch gekennzeichnet, dass** die Querschnittserweiterung (17) zumindest teilweise im Bereich des Verbindungsstücks (14), vorgesehen ist.

10. Reaktor gemäß mindestens einem der Ansprüche 1 bis 9, **dadurch gekennzeichnet, dass** die Größe des Querschnitts der Querschnittserweiterung (17) zumindest der Größe des Querschnitts des Ringraums (20) entspricht.

11. Reaktor gemäß mindestens einem der Ansprüche 1 bis 10, **dadurch gekennzeichnet, dass** das Reinigungsteil (27) als mindestens ein an der radialen Innenseite des Schlittens (23) und an dessen beiden Enden vorgesehener O-Ring ausgebildet ist.

12. Reinigungsverfahren für einen Reaktor (10) gemäß mindestens einem der Ansprüche 1 bis 11, **dadurch gekennzeichnet,**
- **dass** der Kolben (26) eines jeden Schlittens (23) im Züchtungsbetrieb bei Nenndrehzahl der Pumpe (102) in der einen Drehrichtung der Pumpe (102) in einer ersten Fließrichtung (18a) der Kultur im Bereich der Querschnittserweiterung (17) in seiner einen Endlage, nämlich am ersten Anschlag (24) angeordnet ist (Fig. 2 und Fig. 3),
- **dass** bei Reversierung der Drehrichtung der Pumpe (102), unter Bewirkung einer zur ersten Fließrichtung (18a) reversierten oder umgekehrten zweiten Fließrichtung (18b) der Kolben (26) eines jeden Schlittens (23) bei dessen Stillstand von seinem ersten Anschlag (24) zur Anlage an seinem zweiten Anschlag (25) verschoben wird,
- wobei danach zwecks Reinigung jeder Schlitten (23) zusammen mit seinem Kolben (26) in der zweiten Fließrichtung (18b) der Kultur durch den Ringraum (20) bis zum Endanschlag (22) an der anderen Seite eines jeden Rohrs (11) verschoben wird (Fig. 4 und Fig. 5),
- **dass** nach erneuter Umkehr der Fließrichtung (18a, 18b) alle Kolben (26) von der Anlage am zweiten Anschlag (25) zur Anlage am ersten Anschlag (24) verschoben werden und
- **dass** danach die Schlitten (23) zusammen mit den Kolben (26) durch den Ringraum (20), insbesondere unter erneuter Reinigung, bis zum Erreichen des Endanschlags (22) am anderen Ende eines jeden Rohrs (11) in die Ausgangslage zurück bewegt werden.

13. Verfahren gemäß Anspruch 12, **dadurch gekennzeichnet, dass**
- bei Reversierung der Drehrichtung der Pumpe (102) und/oder
- bei Umkehr der Fließrichtung (18a, 18b)
die Drehzahl der Pumpe (102) erhöht wird.

14. Verfahren gemäß Anspruch 13, **dadurch gekennzeichnet, dass** nach Erreichen des Endanschlags (22) die erhöhte Drehzahl der Pumpe (102) wieder auf die die erste, insbesondere normale, Fließrichtung (18a) und eine normale Strömungsgeschwindigkeit bewirkende Nenndrehzahl abgesenkt wird.

15. Verwendung einer Querschnittserweiterung (17) mit einem längs des Außenmantels (30) bewegbaren, kraftschlüssig mit dem Reinigungsteil (27) verbundenen Schlitten (23) in jedem Rohr (11), welcher Schlitten (23) mit einem auf seinem Mantel zwischen zwei Anschlägen (24, 25) verschiebbaren Kolben (23) versehen ist, für einen Reaktor (10) gemäß mindestens einem der Ansprüche 1 bis 11.

## Claims

1. Reactor (10) for photochemical growing of a liquid culture of small organisms and microorganisms, particularly algae, with a tank (101), with an inlet (103) and an outlet (104) connected thereto via a pump (102), wherein the pump (102) rotates in operation with a settable nominal number of rotations in a rotational direction (18a) causing the flow direction (18a) of the culture, and with multiple pipes (11) connected to one another in pairs in succession via connection pieces (14), which multiple pipes (11) are fed in operation from the inlet (103) in flow direction (18a) towards the outlet (104), wherein, for the photochemical growing, the culture is exposed to the light radiation of a rod-shaped lamp (19) having an outer shell (30) which is arranged symmetrically to and inside each pipe (11), by forming an annular space (20), and wherein a cleaning element (27) which cleaning element (27) is shiftable along the outer shell (30) is arranged in each pipe (11) for cleaning it and the radial interior of which cleaning element (27) is adapted to the outer shell (30) in terms of shape and dimension, **characterized in that**
- both ends of each pipe (11) and/or the connection pieces (14) have a cross-section extension (17) with respect to the annular space (20),
- each pipe (11) has a carriage (23) which is movable along the outer shell (30),
- an end abutment (22) is provided in the area of the cross-section extension (17) for each carriage (23),
- each carriage (23) is connected in a force-fitting way with the cleaning element (27),
- the carriage (23) has a shiftable plunger (26) and
- the plunger (26) fills the annular space (20) in the area outside the cross-section extension (17).

2. Reactor according to claim 1, **characterized in that** the end abutment (22) is formed as a plate attached to the connection piece (14) and/or to the pipe (11) and extending into the motion path of the plunger (26).

3. Reactor according to claim 1 or 2, **characterized in that** each carriage (23) is movable on its shell between a first abutment (24) and a second abutment (25).

4. Reactor according to claim 3, **characterized in that** the first abutment (24) and/or the second abutment (25) is formed as projection protruding radially outwards.

5. Reactor according to claim 3 or 4, **characterized in that** the first abutment (24) is provided at the extremity of each carriage (23) which is closer to the tank (101) and/or the second abutment (25) is provided at the extremity of each carriage (23) which is closer to the pump (102).

6. Reactor according to at least one of the claims 1 to 5, **characterized in that** the carriage (23) is adapted to the outer shell (30) at its inner section in terms of shape and dimension.

7. Reactor according to at least one of the claims 1 to 6, **characterized in that** the plunger (26) is formed as ring washer.

8. Reactor according to at least one of the claims 1 to 7, **characterized in that** the plunger (26) is adapted to the annular space (20) in terms of shape and dimension for the filling of the latter.

9. Reactor according to at least one of the claims 1 to 8, **characterized in that** the cross-section extension (17) is provided at least partially in the area of the connection piece (17).

10. Reactor according to at least one of the claims 1 to 9, **characterized in that** the size of the cross-section of the cross-section extension (17) corresponds at least to the size of the cross-section of the annular space (20).

11. Reactor according to at least one of the claims 1 to 10, **characterized in that** the cleaning element (27) is formed as at least an O-ring provided at the radial interior of the carriage (23) and at its both ends.

12. Method for cleaning a reactor (10) according to at least one of the claims 1 to 11, **characterized**
**in that** in growing mode, in case of nominal number of rotations, the plunger (26) of each carriage (23) is arranged in the rotational direction of the pump (102) in a first flow direction (18a) of the culture in the area of the cross-section extension (17) in its end position, that is at the first abutment (24) (Fig. 2 and Fig. 3),
**in that** in case of reversing the rotational direction of the pump (102) by causing a second flow direction (18b) which is reversed or opposite of the first flow direction (18a), the plunger (26) of each carriage (23) is shifted from its first abutment (24) for positioning it at its second abutment (25) during idle mode of the carriage,
wherein thereafter, for the purpose of cleaning, each carriage (23) is shifted together with its plunger (26) in the second flow direction (18b) of the culture through the annular space (20) up to the end abutment (22) on the other side of each pipe (11) (Fig. 4 and Fig. 5),
**in that** after again reversing the flow direction (18a, 18b) all plungers (26) are shifted from the position at the second abutment (25) to the position at the first abutment (24) and
**in that** thereafter the carriages (23) are moved back together with the plunger (26) in the initial position through the annular space (20), particularly subjected to a new cleaning process, until the end abutment (22) is reached at the other end of each pipe (11).

13. Method according to claim 12, **characterized in that** the number of rotations of the pump (102) is increased when the rotation direction of the pump (102) is reversed and/or when the flow direction (18a, 18b) is reversed.

14. Method according to claim 13, **characterized in that** after reaching the end abutment (22) the increased number of rotations of the pump (102) is again reduced to the nominal number of rotations which effects the first, particularly normal, flow direction (18a) and a normal flow speed.

15. Use of a cross-section extension (17) in each pipe (11), with a carriage (23) movable along the outer shell (30) and connected to the cleaning element (27) in a force-fitting way, the carriage (23) being provided with a plunger (23) which is shiftable on its shell between two abutments (24, 25), for a reactor according to at least one of the claims 1 to 11.

## Revendications

1. Réacteur (10) pour cultiver de manière photochimique une culture liquide de petits organismes et microorganismes, particulièrement des algues, avec un récipient (101), avec une entrée (103) et une sortie (104) connectée avec l'entrée par une pompe (102), la pompe (102) tournant en opération avec un ajustable nombre de tours dans une direction de rotation (18a) qui cause la direction d'écoulement (18a) de la culture, et avec des multiples conduits (11) connectés l'un à l'autre en pairs en succession par des pièces de connexion (14), qui sont alimentés en opération en direction d'écoulement (18a) vers la sortie (104), la culture étant exposée, pour la culture photochimique, à la radiation de lumière d'une lampe (19) en forme de bâton ayant une enveloppe extérieure (30) arrangée de manière symétrique par rapport à et à l'intérieur de chaque conduit (11), en formant un espace annulaire (20), et un élément de nettoyage (27) qui est déplaçable le long de l'enveloppe extérieure (30) étant arrangé dans chaque conduit (11) pour le nettoyer et l'intérieur radial duquel étant adapté à l'enveloppe extérieure (30) par rapport à la forme et aux dimensions,
**caractérisé en ce que**
- les deux extrémités de chaque conduit (11) et/ou les pièces de connexion (14) ont une extension de coupe transversale (17) par rapport à l'espace annulaire (20),
- chaque conduit (11) a un chariot (23) qui est mobile le long de l'enveloppe extérieure (30),
- une butée d'extrémité (22) est prévue dans la région de l'extension de coupe transversale (17) pour chaque chariot (23),
- chaque chariot (23) est connecté par fixation à force avec l'élément de nettoyage (27),
- le chariot (23) a un piston (26) déplaçable et
- le piston (26) remplie l'espace annulaire (20) dans la région hors de l'extension de coupe transversale (17).

2. Réacteur selon la revendication 1 **caractérisé en ce que** la butée d'extrémité (22) est formée comme plaque attachée à la pièce de connexion (14) et/ou au conduit (11) et s'étendant dans le chemin de mouvement du piston (26).

3. Réacteur selon la revendication 1 ou 2, **caractérisé en ce que** chaque chariot (23) est mobile sur son enveloppe entre une première butée (24) et une deuxième butée (25).

4. Réacteur selon la revendication 3, **caractérisé en ce que** la première butée (24) et/ou la deuxième butée (25) est formée comme projection qui fait saille radialement vers l'extérieur.

5. Réacteur selon la revendication 3 ou 4, **caractérisé en ce que** la première butée (24) est prévue à l'extrémité de chaque chariot (23) qui est plus proche du récipient (101) et/ou la deuxième butée (25) est prévue à l'extrémité de chaque chariot (23) qui est plus proche de la pompe (102).

6. Réacteur selon au moins l'une des revendications 1 à 5, **caractérisé en ce que** le chariot (23) est adapté à l'enveloppe extérieure (30) à son intérieur par rapport à la forme et les dimensions.

7. Réacteur selon au moins l'une des revendications 1 à 6, **caractérisé en ce que** le piston (26) est formé comme rondelle annulaire.

8. Réacteur selon au moins l'une des revendications 1 à 7, **caractérisé en ce que** le piston (26) est adapté à l'espace annulaire (20) par rapport à la forme et les dimensions afin de remplir l'espace annulaire.

9. Réacteur selon au moins l'une des revendications 1 à 8, **caractérisé en ce que** l'extension de coupe transversale (17) est au moins partiellement dans la région de la pièce de connexion (17).

10. Réacteur selon au moins l'une des revendications 1 à 9, **caractérisé en ce que** les dimensions de la coupe transversale de l'extension de coupe transversale (17) correspond au moins aux dimensions de la coupe transversale de l'espace annulaire (20).

11. Réacteur selon au moins l'une des revendications 1 à 10, **caractérisé en ce que** l'élément de nettoyage (27) est formé comme au moins un joint torique prévu à l'intérieur radial du chariot (23) et à ses deux extrémités.

12. Procédé de nettoyage d'un réacteur (10) selon au moins l'une des revendications 1 à 11, **caractérisé**
**en ce que** dans le mode de culture, en cas du nombre de tours nominal, le piston (26) de chaque chariot (23) est arrangé dans la direction de rotation de la pompe (102) dans une première direction d'écoulement (18a) de la culture dans la région de l'extension de coupe transversale (17) dans sa position finale, c'est-à-dire à la première butée (24) (Fig. 2 et Fig. 3),
**en ce qu'**en cas de l'inversion de la direction de rotation de la pompe (102) en causant une deuxième direction d'écoulement (18b) qui est inversée ou opposée par rapport à la première direction d'écoulement (18a), le piston (26) de chaque chariot (23) est déplacé de sa première butée (24) pour le positionner à sa deuxième butée (25) pendant le mode inactif du chariot,
ensuite, pour le nettoyage, chaque chariot (23) étant déplacé ensemble avec son piston (26) dans la deuxième direction d'écoulement (18b) de la culture à travers l'espace annulaire (20) jusqu'à la butée d'extrémité (22) de l'autre côté de chaque conduit (11) (Fig. 4 et Fig. 5),
**en ce qu'**après une nouvelle inversion de la direction d'écoulement (18a, 18b) tous les pistons (26) sont déplacés de la position à la deuxième butée (25) à la position à la première butée (24), et
**en ce qu'**ensuite les chariots (23) sont retournés ensemble avec le piston (26) dans la position initiale à travers l'espace annulaire (20), particulièrement pendant un nouveau nettoyage, jusqu'à la butée d'extrémité (22) est rejointe à l'autre côté de chaque conduit (11).

13. Procédé selon la revendication 12, **caractérisé en ce que** le nombre de tours de la pompe (102) est augmenté quand la direction de rotation de la pompe (102) est inversée et/ou quand la direction d'écoulement (18a, 18b) est inversée.

14. Procédé selon la revendication 13, **caractérisé en ce qu'**après avoir rejoint la butée d'extrémité (22), le nombre de tours augmenté de la pompe (102) est de nouveau réduit au nombre de tours nominal qui cause la première, particulièrement normale, direction d'écoulement (18a) et une vitesse d'écoulement normale.

15. Utilisation d'une extension de coupe transversale (17) dans chaque conduit (11), avec un chariot (23) mobile le long de l'enveloppe extérieure (30) et connecté par force à l'élément de nettoyage (27), le chariot (23) ayant un piston (23) qui est déplaçable sur son enveloppe entre deux butées (24, 25), pour un réacteur selon au moins une des revendications 1 à 11.
